# EUROPEAN PATENT SPECIFICATION

(11) **EP 2 475 405 B1**
(45) Date of publication and mention of the grant of the patent: **13.04.2016**
(21) Application number: 10754633.5
(22) Date of filing: 09.09.2010
(51) Int. Cl.: A61L 29/04, A61L 29/14, A61L 29/08

(54) **SUBSTRATE SURFACE MODIFICATION UTILIZING A DENSIFIED FLUID AND A SURFACE MODIFIER**
SUBSTRATOBERFLÄCHENMODIFIZIERUNG MIT EINER VERDICHTETEN FLÜSSIGKEIT UND EINEM OBERFLÄCHENMODIFIZIERER
MODIFICATION DE SURFACE DE SUBSTRAT EN UTILISANT UN FLUIDE DENSIFIÉ ET UN MODIFICATEUR DE SURFACE

(30) Priority: 09.09.2009 US 240742 P
(43) Date of publication of application: 18.07.2012
(73) Proprietor: Cook Medical Technologies LLC, Bloomington, IN 47402 (US)
(72) Inventor: LIU, Jian-Lin, Bloomington IN 47401 (US); DEMARS, Bruce, J., Bloomington IN 47401 (US)
(74) Representative: Garratt, Peter Douglas
(86) International application number: PCT/US2010/048268
(87) International publication number: WO 2011/031857

(56) References cited:
- WO-A1-2008/052568
- US-A- 5 968 654
- US-A- 6 106 505
- US-A1- 2002 025 384
- POWELL ET AL: "Chemotherapeutic implants via subcritical CO2 modification", BIOMATERIALS, ELSEVIER SCIENCE PUBLISHERS BV., BARKING, GB, vol. 28, no. 36, 22 October 2007 (2007-10-22), pages 5562-5569, XP022308855, ISSN: 0142-9612, DOI: DOI:10.1016/J.BIOMATERIALS.2007.09.004
- DATABASE WPI Derwent Publications Ltd., London, GB; AN 1997-196403 & JP H0 951941 A

## Description

### BACKGROUND

Technical Field. The present application relates generally to methods for modifying a substrate by exposing the substrate to a densified fluid under controlled conditions, in order to enhance selected properties of the substrate. More particularly, the application relates to method for modifying a polymer by exposing the polymer to densified CO₂ under subcritical and/or supercritical conditions and impregnating the substrate with a surface modifying compound.

Background Information. In the recent past, densified fluid technology has been the focus of numerous research and development studies based on the fundamentals of high-pressure phase behavior of materials. The technique has been well developed, both in fundamental understanding and numerous commercial applications since at least the late 1980s.

A densified fluid, as referred to herein, is a fluid maintained in the vicinity of the critical point of the fluid. Due to their liquid-like solvation power and gas-like mass transfer properties, these fluids are unique solvents for uses such as synthesis, extraction and separation. Processes utilizing such solvents may generally be carried out at moderate temperatures, thereby making the processes suitable for use with many heat-sensitive compounds. In addition, the products resulting from such processes are generally free of residual solvents. This feature is particularly beneficial in, e.g., the medical device fields, due to strict limits on the residual solvent content of the resulting products.

The critical temperature of a compound is defined as the temperature above which the pure gaseous component cannot be liquefied regardless of the pressure applied, while the critical pressure is defined as the gas or vapor pressure at the corresponding critical temperature. The temperature and pressure at which the gas and liquid phases become indistinguishable is known as the critical point. If the conditions exceed the critical points, the densified fluid exists as a supercritical fluid (SCF). If either, or both, of the temperature and pressure of the fluid are slightly below the critical points, and the densified fluid remains in a fluid state and denser than a typical gas, the densified fluid exists in a state referred to herein as a subcritical fluid (SBCF). In some cases, a subcritical fluid is at about 95% or more of its critical temperature and/or 95% or more of its critical pressure. In other cases, either, or both, of the critical temperature and critical pressure may vary by a greater amount from the respective critical temperature or pressure of the fluid. A densified fluid as referred to herein may comprise a fluid existing as either a supercritical fluid or a subcritical fluid.
In the SCF region, the physical properties of a substance are remarkable and intermediate to both liquid and gas. Near the critical point of a fluid, changes in pressure or temperature may significantly alter the physical-chemical properties (e.g., density, diffusivity, or solubility characteristics) of the densified fluid. This can be particularly important in instances in which processing conditions are sensitively manipulated, such as in drug processing. Although many compounds are capable of existing as a SCF or a SBCF, carbon dioxide is a particularly attractive compound for such use. Carbon dioxide is inexpensive, and poses little threat to the environment or human health in the amounts used as a SCF or a SBCF (e.g., as SCCO₂ or SBCCO₂). Other fluids that are capable of use as a SCF or SBCF in various applications include methane, ethane, propane, argon, nitrous oxide and water. These fluids have been utilized as supercritical fluids for applications as diverse as extraction, inorganic/organic synthesis, catalysis, material processing, and even dry-cleaning.

It is desired to utilize densified fluid technology in a manner such that the properties of a substrate, such as a polymer, may be modified in order to enhance selected properties of the substrate. It is further desired to utilize densified fluid technology to modify selected properties of a substrate in a manner that does not require the use of organic solvents. It is further desired to utilize densified fluid technology to impregnate a substrate, e.g., with one or more surface modifying compounds.

Reference is directed to WO 2008/052568 which discloses a method of producing an article comprising an interpenetrating polymer network (IPN). A polymer substrate e.g. shaped to provide the desired article is exposed in a reaction chamber to at least one monomer for an interpenetrating polymer in the presence of an impregnation solvent comprising CO₂ under conditions wherein the CO₂ is in its liquid or supercritical state. The monomers may be fluorinated and may contain silicone. If silicone rubber is used at the polymer substrate, new articles such as catheters may be produced. Reference is also directed to US 5,968,654 which discloses a method of modifying a polymeric substrate to impart water-repellency and lubricity and provide the additional benefit of reducing the soiling of fabrics. The method includes combining the polymeric substrate at a dense or liquified gas such as carbon dioxide or sulfur hexafluoride and a fluorinated compound such as a fluorinated polyether. Reference is further directed to US 2002/0025384 which discloses a method of treating a substrate comprising contacting a surface of the substrate with a pressurized fluid comprising carbon dioxide and a surface treatment component. The contacting step is preferably carried out by immersion, the fluid is preferably a liquid or supercritical fluid and the surface treatment component may be a fluoracrylate polymer. Reference is further directed to US6106505, which discloses polymeric medical articles comprising the antiinfective agents chlorhexidine and triclosan incorporated therein, in combination with hydrophobic polymers, e.g. a silicone polymer. Incorporation of these agents takes place by impregnating the polymeric article with a treatment solution comprising the hydrophobic polymer, chlorhexidine, triclosan and a solvent. Reference is further directed to JPH0951941, which discloses a catheter tubing made of a flexible vinyl chloride resin containing from 1 to 5 wt% of silicone oil. The catheter has an improved lubricity.

### SUMMARY

In certain aspects, the present invention relates to methods for loading surface modifying agent into a polymeric substrate utilizing densified fluid as a carrier for the agent. The polymeric substrate is a polymeric catheter tubing, and can be comprised of an elastomer, such as a thermoplastic polyurethane elastomer or nylon elastomer. All or a portion of the substrate can be treated with the surface modifying agent. The surface modifying agent is a silicone and/or fluorine-containing compound and serves to reduce the surface tension of a surface of the substrate, by causing a significant increase in the contact angle of a water droplet received on the surface as compared to a corresponding unmodified substrate.

Further embodiments of the invention provide unique catheters having polymeric catheter tubing with an external surface, wherein the surface tension of the external surface has been modified by incorporation of a surface modifying agent into the polymeric catheter tubing. The polymeric catheter tubing can be comprised of an elastomer, such as a thermoplastic polyurethane elastomer or nylon elastomer. All or a portion of the tubing can be treated with the surface modifying agent. The surface modifying agent, which is a silicone and/or fluorine-containing compound, serves to reduce the surface tension of the surface of the catheter, by causing a significant increase in the contact angle of a water droplet received on the surface as compared to a corresponding unmodified substrate. The treated catheter tubing can be configured in the catheter for implantation into the patient, e.g. to reside in a blood vessel, and/or can serve as a catheter extension to reside external of the patient while providing fluid communication with an implanted segment of catheter tubing.

Still further embodiments of the invention, as well as features and advantages thereof, will be apparent to those of ordinary skill in the art from the descriptions herein.

### BRIEF DESCRIPTION OF THE DRAWINGS

Figure 1 is a schematic diagram of an experimental setup for modification of a substrate utilizing densified fluid technology.
Figure 2 is a digital image of a photograph of a droplet of distilled water received on an untreated thermoplastic polyurethane elastomer control film, bearing markings for estimate of the contact angle between the outer edge of the droplet and the film.
Figure 3 is a digital image of a photograph of a droplet of distilled water received on a thermoplastic polyurethane elastomer film corresponding to that shown in Figure 2 but having been treated for surface modification according to an aspect of the invention, bearing markings for estimate of the contact angle between the outer edge of the droplet and the film.
Figure 4 provides an illustration of one embodiment of a catheter in accordance with the invention.
Figure 5 provides a cross-sectional view of co-extruded catheter tubing that can be treated in accordance with the invention.

### DETAILED DESCRIPTION

For purposes of promoting an understanding of the present invention, reference will be made to certain embodiments thereof, and specific language will be used to describe the same. It should nevertheless be understood that no limitation of the scope of the invention is thereby intended, such alterations and further modifications in the described embodiments, and such further applications of the principles of the invention as illustrated herein being contemplated as would normally occur to one skilled in the art to which the invention relates.

The present patent application sets forth multiple applications for which densified fluid technology may be utilized. The following discussion will primarily describe such use in connection with medical devices. Those skilled in the art will recognize, however, that the scope of the invention includes applications of the technology other than in connection with such devices.

A feature of the present invention relates to the modification of the surface of a substrate utilizing densified fluid technology, in a manner such that the chemical and/or physical properties of the substrate surface may be altered in a controlled fashion. The substrate comprises a polymer, and the polymer is modified by incorporation of an agent capable of enhancing the surface properties of the polymer. The agent is a fluorinated molecule or a silicone-containing molecule, and the substrate may be an elastomeric polymer, and the agent is effective to reduce the surface tension of the polymer, e.g. so as to increase the lubricity of the surface or reduce the tackiness or thrombogenicity of the surface.

Densified fluids, such as densified CO₂, are free of hazardous solvents, making them a favorable alternative in place of organic solvents. Densified CO₂ is a particularly preferred choice for use in the processing of the invention. When exposed to a densified fluid, such as densified CO₂, polymers exhibit various extents of swelling and enhanced chain mobility. Optimization of such factors, e.g., controlling the pressure and temperature at which the polymer is exposed to the densified fluid, may significantly facilitate and accelerate the ability of the polymer to receive a solute, such as a surface modifying agent, from the densified fluid solvent.

When impregnating a compound into a medical device or other matrix utilizing a densified fluid solvent, the impregnation process can include the following general steps. Initially, one or more substrates intended for impregnation are positioned within a suitable reactor. The reactor should be of a type capable of withstanding pressures in a supercritical range. The compound to be impregnated into the substrate, such as a surface modification agent, is then introduced, e.g. via injection, into the reactor. The densified fluid, such as densified CO₂, is then introduced into the reactor under conditions such that the modifier compound is initially dispersed or dissolved in the fluid, and thereafter transfers from the densified fluid to the matrix of the polymer, including at least regions of the polymer matrix bounding the surface of the substrate. The densified fluid is then released from the reactor in a rapid, yet controlled, manner, such that the modifier compound remains trapped within the polymer matrix. As an alternative embodiment, the densified fluid may be introduced prior to the introduction of the modifier compound.

If desired, the fluid may be introduced into the reactor as a SCF, and the entire impregnation may be carried out at supercritical conditions. Typical supercritical conditions for SCCO₂ are about 40°C and 28 MPa (4000 psi), and a typical period of exposure is 1-2 hours. To facilitate the impregnation process, a substrate having a low Tg will promote loading of the modifier compound. This is particularly true for elastomeric polymers such as poly(ether block urethane), poly(ether block amide), rubbers, and the like. On the other hand, due to the relatively high rigidity of its polymer backbone, a substrate with a high Tg would undergo a slower impregnation. In addition, when the substrate is blended with a large amount of inorganic material, such as BaSO₄ or BiOCl, the rate or extent of impregnation with the surface modifying agent may also be impacted. In certain embodiments, the substrate will contain significant amounts of radiopaque particulate substances, such as BaSO₄ or BiOCl, for example wherein the substrate is constituted at least 10% by weight of the radiopaque substance, commonly from about 10% to about 50% by weight. Catheter tubing substrates so loaded with radiopaque substances are preferred substrates herein.

On some occasions, it may be preferred to at least initially introduce the densified CO₂ into the reactor at subcritical conditions, e.g., at about 4.8 - 5.5 MPa (about 700-800 psi) and 20-25°C. Impregnation at subcritical conditions is less aggressive than at supercritical conditions. This can affect both the solubility of modifier compound and the substrate polymer in the subcritical condition. Although impregnation at subcritical conditions can take longer than at supercritical conditions, the impregnation can be used and may provide certain advantages. Subcritical CO₂ can be less aggressive toward the substrate and the modifier compound. In certain embodiments, the modifier compound will be fully impregnated to a desired loading solely under subcritical CO₂ conditions. In such cases, there is no need to increase the conditions to the more severe supercritical state. In other cases, following exposure at the subcritical conditions, the reactor can be adjusted to supercritical conditions, if desired, to complete the impregnation. On these occasions, impregnation of the surface modifier compound can be carried out by exposure to the substrate at the subcritical conditions for a set period of time (such as 1-2 hours), and thereafter adjusting the conditions to the supercritical range.

Due to the partial solubility of some modifier compounds in water, it is possible that the compounds may have a higher elution rate from the impregnated polymer than desired. In certain desirable embodiments, essentially no elution will desired when the polymer substrate is contacted with aqueous mediums such as water or those which occur in biological systems. Any partial solubility of a modifier compound in water may be countered to some extent by increasing/enhancing the compound's interaction with the polymer substrate. One way to improve this interaction is to increase the molecular dispersion of the compound into the substrate. In this case, hydrogen bonds or other non-covalent interactions can form between the compound and the substrate. These bonds or other interactions allow the surface modifying agent to be held much more firmly in the substrate, and therefore provide slowed, minimal or essentially no release of the modifier compound when exposed to water or other aqueous environments.

The duration of the reaction may vary depending upon the type of polymer exposed to a given SBCF or SCF. For example, for a generally rigid polymer, such as a polyester, a longer time is favorable. For a softer grade polymer, such as a polyurethane elastomer or nylon elastomer, the reaction time can generally be shorter. Similarly, for a particular polymer (e.g., a polyurethane), a higher durometer grade of that polymer will typically be exposed to the densified fluid for a longer time than a lower durometer grade, other factors remaining the same. Generally speaking, a polymeric substrate with a lower Tg, or having a higher amorphous portion, would be favored for this process, as it is more amenable to loading under a particular set of conditions than would a substrate with a higher Tg, or having a lower amorphous portion.

In certain embodiments, the impregnation with the surface modifier can be carried out using a dual reactor system. In this system, first and second reactors are operationally linked, and separated by a valve system that allows selective communication between the two reactors. When utilizing a dual reactor system, the modifier compound can be placed in the first reactor. Although typically both reactors will be capable of withstanding supercritical conditions, this need not necessarily be the case, and the first reactor need only be capable of withstanding subcritical conditions when those are intended for use. The substrates for impregnation are placed in the second reactor, which must be capable of withstanding the intended conditions, typically supercritical conditions. Liquid CO₂ is initially introduced into the first reactor through a pump. Illustratively, the temperature of the first reactor can initially be set at 20-25°C, and the pressure set at about 4.8 - 5.5 MPa (700-800 psi). Those skilled in the art will appreciate that other suitable conditions may be substituted for those listed. An initial reaction is carried out in the first reactor for a suitable period of time, e.g., about 1-2 hours. During exposure to the SBCCO₂ or SCCO₂ in the first reactor, the modifier compound can become well dispersed or dissolved in the densified CO₂.

Following generation of the modifier-loaded fluid in the first reactor, a valve between the reactors is opened, and the contents of the first reactor are pumped into the second reactor. The second reactor can be maintained at supercritical conditions, such as at about 40°C and 28 MPa (4000 psi) for CO₂. By exposing the polymer substrate to the modifier compound dispersed or dissolved in the densified fluid for an appropriate period of time, e.g., for about 1-2 hours, higher loadings (e.g. higher concentrations and/or deeper penetration) of the surface modifier into the polymer matrix may be achieved.

The selection of subcritical and/or supercritical conditions for impregnation may be varied as desired. Among the factors that may be considered when determining the conditions for a particular impregnation are the respective properties of the modifier compound, the particular substrate to be impregnated, the chemical structure and particle size or liquid nature of the modifier compound, as well as the desired degree of impregnation of the modifier compound into the substrate.

The swelling extent and alteration of the microstructure of the polymer upon exposure to a densified fluid, such as subcritical or supercritical CO₂, depends on the chemical nature of the polymer and its ability to interact with the densified fluid. A substrate having a lesser ability to swell will generally be more difficult to impregnate with the modifier compound than a substrate having an increased ability to swell. Thus, as stated above, highly crystalline polymers, such as PTFE, are generally of lesser suitability as an impregnating matrix due to the limited ability of these polymers to swell upon exposure to a densified fluid. On the other hand, less crystalline polymers, such as polyurethane, and/or polymers with large amorphous fractions, are more capable of swelling upon exposure to the densified fluid. Therefore, these compounds are generally of greater suitability as an impregnating matrix. Generally speaking, it is expected that modifier compound infusion into the polymer matrix will be more pronounced in a rubbery and semi-crystalline polymer than in a crystalline one.

Fluorinated molecules and silicone-containing molecules are the compounds used in surface modification according to the present invention. Fluorinated and silicone-containing molecules have very low surface tension properties and dielectric constants as compared to organic molecules sometimes utilized for such purposes. Compounds containing fluorine and silicone are in widespread use in the medical device arts due to their favorable chemical resistance and biocompatibility.
Various methods have been used in the past to impregnate or bond these two molecules onto the surface of a substrate to modify its surface properties. These prior art methods have generally either required that the compound be initially dissolved in an organic solvent, or alternatively, have required cumbersome techniques and/or expensive equipment (e.g., electrical chemical deposition). On the other hand, modifying the surface by dispersing the agent (e.g., the fluorine or silicone-containing compound) in a densified fluid, and exposing the substrate to the densified fluid under controlled conditions provides a more convenient and secure manner of impregnation. In addition, surface modification in this manner minimizes the unintended migration of the subject compound from the substrate surface.

Surface modification of a polymeric substrate may be carried out on a nanoscale by controlling the impregnation process utilizing a densified fluid, such as supercritical CO₂. The fluorinated or silicone-containing molecules are dissolved or dispersed in the densified fluid, and the polymeric substrate is then exposed to the densified fluid under supercritical conditions. Although an initial exposure under subcritical conditions may be helpful in a particular case, it is believed that exposure under supercritical conditions will provide better surface modification.

Reaction conditions may be selected such that fluorinated or silicone compound may be controllably anchored over a reasonable depth in a polymer surface. When the impregnating compound has an unusually high surface tension, a suitable surface modifier may be utilized to reduce the surface tension of the impregnated substrate to a degree that enables it to be suitable for broad applications (e.g., anti-thrombogenics). The degree of impregnation can be controlled, so that the surface properties of the polymer substrate can be modified as desired.

As noted above, relatively soft polymeric substrate materials can be used in processes and products of the invention, and have been discovered to lend themselves to effective incorporation of and surface modification by modifier compounds when carried by densified fluids such as densified CO₂. In some forms, a polymeric material used in the invention can have a Shore A hardness (ASTM D2240) of less than about 100, for example in the range of about 40 to about 100, or within the range of about 50 to about 95. Some advantageous embodiments employ polymeric materials with a Shore A hardness in the range of about 70 to about 90. Polymeric elastomers having such hardness values, and particularly thermoplastic polyurethane elastomers (e.g. block copolymers as mentioned herein) and nylon elastomers, are used in certain embodiments.

Particularly preferred examples of polymers suitable for impregnation via this technique include block copolymers, such as a polyether block amide copolymer (e.g., PEBAX®), and a polyurethane block copolymer. Other nylon elastomers are also preferred. These block copolymers have amide and urethane functional groups that provide them with a relatively high degree of hydrophilic properties (water-like). However, this property otherwise hinders the introduction of fluorinated and silicone compounds into the matrix of such polymers by conventional processes. In addition, once introduced by such conventional processes, the compounds have a tendency to migrate out of the substrate due to the incompatibility between the compound and the substrate.

In addition to the copolymers listed above, those skilled in the art will appreciate that the class of polymer substrates that may undergo surface modification upon exposure to densified fluid in this manner is virtually unlimited. For example, semi-crystalline polymers, such as nylon 12, can be impregnated effectively. The densified fluid will induce the transition of the polymer surface into a rubbery phase which facilitates the impregnation of molecules. When the densified fluid (e.g., SCCO₂ or SBCCO₂) is released, the polymer surface will return to glassy state, which helps the surface retention of molecule.

It is believed that the degree of solubility of the agent in the densified fluid enhances the ability of the agent to be incorporated into the surface of the polymer. Therefore, in one particularly preferred embodiment of the present invention, fluorinated compounds and silicones are functionalized by incorporating relatively small portions of one or more functional groups to enhance the solubility of the agent in the densified (e.g., supercritical) fluid and/or enhance the compatibility of the compounds with the polymer substrate. The main portion of the fluorinated or silicone compounds will have molecular weights high enough that both compounds are liquid or solid at room temperature. Either liquid or solid compounds will facilitate the CO₂ assisted impregnation process. The physical state of the compounds can be a significant factor in an impregnation. However, the solubility of the compound in the supercritical fluid (SCCO₂) is typically of more significance in terms of the loadings of the compounds. Thus, lower molecular weight materials are generally preferred over higher molecular weight materials. Silicone oils, including silicone oils functionalized with at least one polar group, such as hydroxyl-functional silicone oils, are used in certain embodiments.

Suitable functional groups include polar compounds, such as hydroxyl, amino, carboxylic acid, nitro, thio, urea, and urethane, among others. The polar groups act as anchors to the substrate, so that the compound won't migrate out of the substrate. Most of the fluorinated and silicone compounds have only limited miscibility with many organic substrates. Preferably, the content of the polar group will not be high enough to negatively affect the solubility within the SCCO₂.

A non-exclusive listing of preferred functionalized fluorine and silicone-containing compounds that may be incorporated into the surface of a substrate includes the compounds listed below:
Functionalized fluorinated compounds; having the formula

   -(CF₂)ₙ-R;

   -(O-CF₂-CF₂)ₙ R; or

   -{CF(CF₃)-CF₂}ₙ-R;

   where:
   n = 1 to about 100, and
   R is - OH;
      - NH₂;
      - NH₂;
      - COOH;
      -COOCH₃; or
      -OCOC(CH₃)=CH₂.
Functionalized Poly(dimethylsiloxanes), having the formula

   -{Sᵢ(CH₃)₂-O}ₙ-Sᵢ(CH₃)₂-R¹

   where
   n=1 to about 100, and
   R¹ is - OH;
      - NH₂;
      - COOH;
      -(CH₂CH₂O)ₘH, where m=1 to about 20;
      -OCOC(CH₃)=CH₂; or
      -(CH₂)ₚO(CH₂)qOH, where p and q each = 1 to about 6, and can be the same as or different from each other.

One class of silicone compounds that can be used is monocarbinol terminated poly(dimethylsiloxanes), including those having the formula:

HOCH₂CH₂OCH₂CH₂CH₂-Si(CH₃)₂-O-{Si(CH₃)₂-O}ₙ-Si(CH₃)₂-R²

where -R² is an alkyl group, such as a C₁ to C₁₀ alkyl group, including but not limited to methyl, ethyl, n-propyl, iso-propyl, n-butyl, isobutyl, or pentyl.

A typical application for densified fluid surface modification may include modifying the surface of a polymer by introducing, e.g., the functionalized compounds onto the surface of the polymer substrate as described. This action changes the surface chemistry of the polymer, and makes the tubing surface less thrombogenic. In addition, such modification may be carried out to alter the surface chemistry of the polymer, and make the surface less tacky. The surface modification can be conducted sufficiently to reduce the surface tension of the substrate surface, for example resulting in an increase in the contact angle of the surface with a drop of distilled water as compared to a corresponding unmodified substrate. The surface modification can involve the impregnation of the modifying agent into the polymer matrix of the polymer substrate, including at least those portions of the polymer matrix that bound the surface of the substrate. Deeper penetration of the agent is also possible, so long as the surface properties of the substrate remain modified.

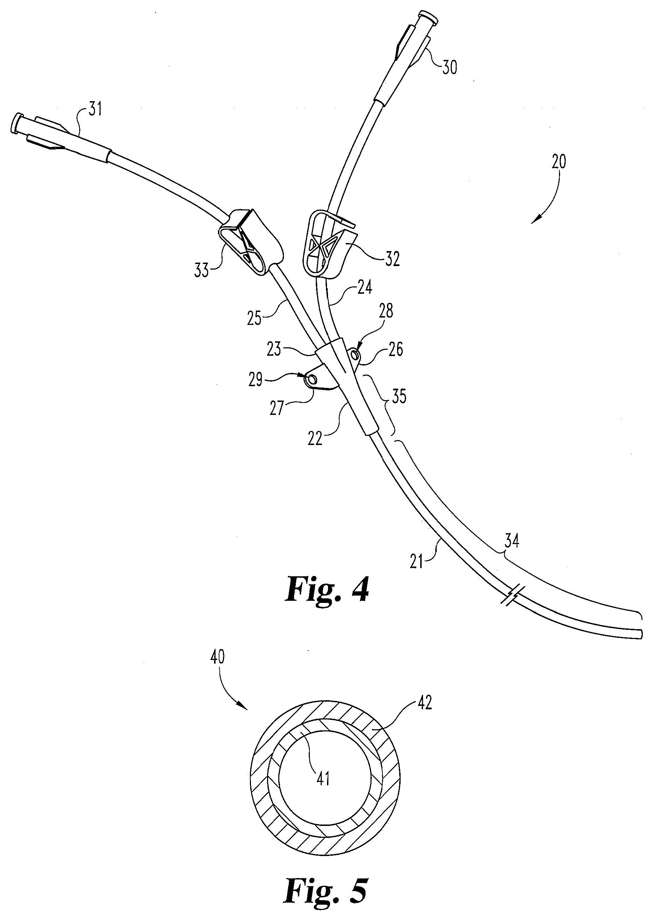

With reference to Figure 4, the treated substrate can be a component of a catheter 20. Catheter 20 has a catheter tubing segment 21 for insertion into the patient. A catheter manifold hub 22 can be bonded to segment 21. Catheter tubing 21 can optionally define multiple inner lumens, and hub 22 can have a proximal end 23 with an equal number of openings (e.g. two, as shown) and be fitted with proximal catheter extensions 24 and 25 fluidly coupled to the respective inner lumens of tubing 21 through hub 22. Hub 22 can have laterally-extending members 26 and 27 having respective holes 28 and 29 for securing the hub 22 to skin of the patient, for example using sutures or staples, to secure the position of the catheter 20 when the segment 21 is implanted in the patient. Catheter 20 in certain embodiments is a central venous catheter (CVC), optionally adapted as a peripherally inserted central venous catheter (PICC). Catheter 20 can also be equipped with terminal hubs 30 and 31 bonded to extensions 24 and 25, respectively, and clamps 32 and 33 fitted upon extensions 24 and 25, respectively, which can be selectively opened and closed by a user to allow and cut off fluid communication across the position of the clamps 32 and 33 on the extensions. Catheter extensions 24 and 25 can be made from the same material as or a different material from tubing 21.

In some embodiments, segment 21 of catheter 20, or at least a portion thereof, will be treated with a surface modifier in accordance with the invention. Such surface modification can lower the surface tension of the surface of segment 21 and improve biocompatibility, e.g. by reducing the risk of thrombosis caused by catheter 20. In such embodiments, a first longitudinal segment 34 can be surface modified, while a second longitudinal segment 35 proximal thereof, is not. In this fashion, hub 22 can be bonded over segment 21 on an untreated portion, thus avoiding the potential of the surface modifier deleteriously interfering with the bond. The unmodified segment 35 can, for example, be created by masking the segment 35 with a tubular mask element such as a metallic or polymeric tube, and subjecting the resulting construct to surface modification processing as described herein to modify segment 34. The masking element can then be removed for further manufacture of the catheter.

In other embodiments, one or both extensions 24 or 25, or portions thereof, will be treated with a surface modifier in accordance with the invention, in addition to or as an alternative to treatment of segment 21 or portions thereof. Such surface modification can for example improve handling properties of the extension(s), illustratively be reducing the tackiness of their surfaces.

Catheter segments to be implanted (e.g. segment 21) or catheter extensions (e.g. 24 or 25) can be made of soft polymeric materials as described herein, including those having Shore A hardness values within the preferred ranges as specified herein, and in particular embodiments are comprised of elastomers such as polyurethane elastomers or nylon elastomers, including those specific materials as identified herein. Additionally, it will be understood that catheter types other than that depicted in Figure 4 can be processed according to the invention to modify the surface properties of at least portions thereof, particularly tubular portions thereof.

In accordance with some inventive embodiments the substrate to be modified can be comprised of two or more different polymeric materials, one or some of which may be more susceptible to impregnation with the surface modifying compound using the densified fluid. Advantageously, with reference to Figure 5, in one embodiment, co-extruded catheter or other tubing 40 can have an inner polymeric wall material 41 and an outer polymeric wall material 42 that differ from one another. Inner wall material 41 can be a harder polymeric material and outer wall material 42 can be a softer polymeric material, wherein the latter is more susceptible to impregnation with the surface modifier using the densified fluid. The inner wall material 41 can thus contribute important properties, such as pushability, to the overall catheter, while the outer wall material 42 can serve as a receptive reservoir for impregnation with the surface modifying agent. Impregnation with the agent(s) can be conducted, for example, using any of the processing techniques described herein. The resulting tubing 40 can have higher levels of the modifying agent in the outer layer 42 than the inner layer 41. For example, the inner layer 41 can simply have a reduced level of the agent than the outer layer 42, or can be essentially free from the agent, depending on the materials and processing conditions selected. The resulting tubing can for example then be used as tubing segment 21 and/or extension segments 24 and 25 of catheter 20 (Fig. 4). The inner layer 41 and outer layer 42 can both be made from thermoplastic polyurethane elastomer (TPU), such as any of those identified herein, wherein the TPU in layer 41 has a greater hardness (e.g. as measured on the Shore A scale) than the TPU in layer 42. In certain forms, the Shore A hardness for the inner TPU can be about 75 to less than 90, and that of the outer TPU can be about 90 to 100. In addition or alternatively, the TPU of the outer layer can be an aliphatic poly(ether-b-urethane) and the TPU of the inner layer can be an aromatic poly(ether-b-urethane). The mechanical properties can be adjusted, e.g., either from the grade of the materials or the wall thickness of each of the materials.

Amorphous polymers with a glass transition temperature below room temperature are especially preferred for surface modification processes of the invention, although other polymers with a glass transition temperature above room temperature may be useful in a particular application. Non-limiting examples of suitable substrate materials included in this disclosure include elastomeric block copolymers. Specific examples include polyurethane elastomers, nylon elastomers (PEBAX series), polyolefin block copolymers, polyesters, PLA/PLGA and their copolymers, as well as silicones. Non-limiting examples of particularly preferred polymers include segmented block copolymers, such as poly(ether-b-amide) and poly(ether-b-urethane), as well as silicone. A more rigorous condition would typically be needed for those polymers with higher Tg and/or a more crystalline structure.

For the purpose of promoting a further understanding of certain aspects of the invention, the following specific example is provided. It will be understood that this examples is illustrative, and not limiting, of the invention.

### Example 1. Surface Modification of Polymeric Substrate

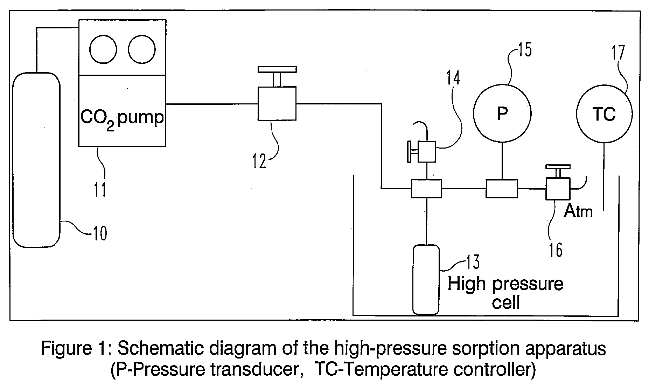

In this example, polyurethane film was impregnated with the surface modifying agent from a densified CO₂ solvent. Figure 1 is a schematic diagram of the experimental setup. A tank 10 of CO₂ fed to a CO₂ pump 11. Pump 11 fed CO₂ through system valve 12 and into a high pressure impregnation cell 13, in which the substrate to be impregnated was positioned. Cell 13 was equipped with a valve 14 for pressure injection of materials into cell 13, if desired. The system included a pressure transducer 15 for monitoring pressure during impregnation, which was vented to atmosphere through valve 16. A temperature controller 17 controlled the temperature of the high pressure cell 13.

The surface modifier utilized was a silicone oil, comprised of a mono-carbinol-terminated polydimethylsiloxane (viscosity 15-20 mm²s⁻¹ (15-20 centistokes), molecular weight approximately 1000). The polyurethane elastomer film was formed with Pellethane 2383-88 (Lubrizol). The film substrate was placed in the high pressure cell 13 along with 2mL of the silicone oil. The substrate was then processed with supercritical CO₂ in the cell 13 at 28 MPa (4000 psi) and 50°C for 1-3 hours. The pressure was released and the sample recovered and rinsed with hexane to remove excess silicone oil resting on the surface of the film.

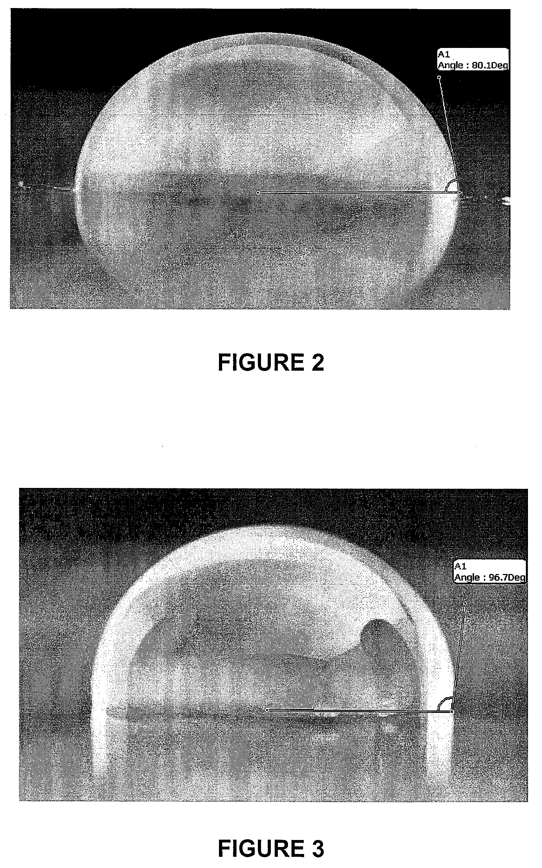

To characterize the effect of the process, the contact angle between a drop of water and the treated film was studied. Photographs were taken both a control film made with the same base material and the corresponding treated film with a similarly-sized small drop of distilled water on the film surface. The contact angle between the edge of the drop and the film was then estimated using commercially available computer software. Figure 2 depicts the control film (which had also been rinsed with hexane), whereas Figure 3 depicts the treated film. The contact angle for the control film was 80.1 Degrees, and the contact angle for the treated film was 96.7 Degrees. Thus, the treatment provided an increase in the contact angle of over 16 Degrees, representing about a 20% increase in the contact angle compared to the untreated control film. It was thus demonstrated that significant decreases in the surface tension of a polymer substrate can be achieved in accordance with the invention.

In additional testing, it was demonstrated that the impregnated silicone oil did not leach from the polymer film when heated in an oven. It was also demonstrated that the silicone oil could be extracted from the treated film upon immersion and soaking in hexane, evidencing that the retention of the silicone oil modifier in the film was not dependent upon covalent bonding.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

One advantage of the use of a densified fluid, such as SCCO₂, as a "temporary" plasticizer in polymer processing lies in the ability of the densified fluid to weakly interact with the backbone in polymers. This leads to a reduction of the Tg, Tm in some polymers, polymer swelling, and the facilitation of solute mass transport within polymer matrices. The specific uses of this technology discussed in this application are not intended to be exclusive, and numerous other uses of this technology are within the scope of the present invention.

Accordingly, while the invention has been illustrated and described in detail in the drawings and foregoing description, the same is to be considered as illustrative and not restrictive in character, it being understood that only certain embodiments have been shown and described and that all changes and modifications that come within the scope of the accompanying claims are desired to be protected.

## Claims

1. A method of modifying the surface properties of a catheter tubing polymeric substrate, comprising:
providing a densified fluid having at least one silicone and/or fluorine-containing compound dispersed or dissolved therein;
providing a polymeric substrate, wherein the polymeric substrate is catheter tubing, wherein the polymer substrate has a surface capable of incorporating the at least one silicone and/or fluorine-containing compound, and wherein incorporation of the at least one silicone and/or fluorine-containing compound into the surface of the polymeric substrate will lower the surface tension of the surface;
positioning the polymeric substrate in a reactor;
introducing into the reactor the densified fluid having the at least one silicone and/or fluorine-containing compound dispersed or dissolved therein;
maintaining the reactor under conditions such that the at least one silicone and/or fluorine-containing compound transfers from the densified fluid into the surface of the polymeric substrate so that the at least one silicone and/or fluorine-containing compound lowers the surface tension of said surface.

2. The method of claim 1, wherein the densified fluid is introduced into the reactor under subcritical conditions; and, wherein the subcritical conditions are maintained in the reactor for a controlled period of time, whereupon the conditions in the reactor are adjusted to supercritical conditions.

3. The method of claim 1, wherein the densified fluid is introduced into the reactor under supercritical conditions.

4. The method of any one of claims 1-3, wherein the silicone and/or fluorine-containing compound includes a polar functional group selected from the group consisting of hydroxyl, amino, carboxylic acid, nitro, thio, urea, and urethane.

5. The method of any one of the preceding claims, wherein the densified fluid comprises densified carbon dioxide.

6. A material obtained or obtainable by any of the methods of claims 1-5.

7. The method of any of claims 1-5 wherein the surface modifying agent is a compound having the formula
-(CF₂)ₙ-R;
-(O-CF₂-CF₂)ₙ-R; or
-{CF(CF₃)-CF₂}ₙ-R;
wherein:
n is equal to 1 to 100, and
R is - OH;
- NH₂;,
- COOH;
-COOCH₃; or
-OCOC(CH₃)=CH₂.

8. The method of any of claims 1-5 wherein the surface modifying agent is a compound having the formula
-{Sᵢ(CH₃)₂-O}ₙ-Sᵢ(CH₃)₂-R¹
wherein:
n=1 to 100, and
R¹ is - OH;
- NH₂;
- COOH;
-(CH₂CH₂O)ₘH, where m=1 to 20;
-OCOC(CH₃)=CH₂; or
-(CH₂)ₚO(CH₂)_{q}OH, where p and q each = 1 to 6, and can be the same as or different from each other.

9. The method of any of claims 1-5 wherein the surface modifying agent is a compound having the formula
HOCH₂CH₂OCH₂CH₂CH₂-Si(CH₃)₂-O-{Si(CH₃)₂-O}ₙ-Si(CH₃)₂-R²
wherein -R² is a C₁ to C₁₀ alkyl group.

## Patentansprüche

1. Verfahren zur Modifizierung der Oberflächeneigenschaften eines Katheterschlauch-Polymersubstrats, umfassend:
Bereitstellen eines verdichteten Fluids, das mindestens eine darin dispergierte oder gelöste Silikon und/oder Fluor enthaltende Verbindung aufweist;
Bereitstellen eines Polymersubstrats, wobei das Polymersubstrat ein Katheterschlauch ist, und das Polymersubstrat eine Oberfläche aufweist, die die mindestens eine Silikon und/oder Fluor enthaltende Verbindung aufnehmen kann, und wobei das Aufnehmen der mindestens einen Silikon und/oder Fluor enthaltenden Verbindung in die Oberfläche des Polymersubstrates die Oberflächenspannung des Polymersubstrats senkt;
Positionieren des Polymersubstrats in einem Reaktor;
Einführen des verdichteten Fluids, das die mindestens eine darin dispergierte oder gelöste Silikon und/oder Fluor enthaltende Verbindung aufweist, in den Reaktor;
Halten des Reaktors unter solchen Bedingungen, dass die mindestens eine Silikon und/oder Fluor enthaltende Verbindung aus dem verdichteten Fluid in die Oberfläche des Polymersubstrats übergeht, so dass die mindestens eine Silikon und/oder Fluor enthaltende Verbindung die Oberflächenspannung der Oberfläche senkt.

2. Verfahren nach Anspruch 1, wobei das verdichtete Fluid unter subkritischen Bedingungen in den Reaktor eingebracht wird; und wobei die subkritischen Bedingungen im Reaktor für einen kontrollierten Zeitraum gehalten werden, woraufhin die Bedingungen in dem Reaktor auf superkritische Bedingungen eingestellt werden.

3. Verfahren nach Anspruch 1, wobei das verdichtete Fluid unter superkritischen Bedingungen in den Reaktor eingebracht wird.

4. Verfahren nach einem der Ansprüche 1 bis 3, wobei die Silikon und/oder Fluor enthaltende Verbindung eine polare funktionelle Gruppe aufweist, ausgewählt aus der Gruppe, bestehend aus Hydroxyl, Amino, Carbonsäure, Nitro, Thio, Harnstoff und Urethan.

5. Verfahren nach einem der vorhergehenden Ansprüche, wobei das verdichtete Fluid verdichtetes Kohlendioxid umfasst.

6. Material, das nach einem der Verfahren nach den Ansprüchen 1 bis 5 erhalten wird, oder sich erhalten lässt.

7. Verfahren nach einem der Ansprüche 1 bis 5, wobei das oberflächenmodifizierende Mittel eine Verbindung der folgenden Formel ist:
**-(CF₂)ₙ-R;**
**-(O-CF₂-CF₂)ₙ-R; oder**
**-{CF(CF₃)-CF₂}ₙ-R;**
wobei:
n gleich 1 bis 100 ist, und
R gleich -OH;
-NH₂;
-COOH;
-COOCH₃; oder
-OCOC (CH₃)=CH₂ ist.

8. Verfahren nach den Ansprüchen 1 bis 5, wobei das oberflächenmodifizierende Mittel eine Verbindung der folgenden Formel ist:
**-{Sᵢ(CH₃)₂-O}ₙ-Sᵢ(CH₃)₂-R¹**
wobei:
n=1 bis 100 ist, und
R¹ gleich -OH;
-NH₂;
-COOH;
-(CH₂CH₂O)ₘH, wobei m=1 bis 20;
-OCOC (CH₃ =CH₂; oder
-(CH₂)ₚO(CH₂)_{q}OH ist, wobei p und q jeweils gleich 1 bis 6 sind, und gleich oder verschieden voneinander sein können.

9. Verfahren nach einem der Ansprüche 1 bis 5, wobei das oberflächenmodifizierende Mittel eine Verbindung der folgenden Formel ist:
**HOCH₂CH₂OCH₂CH₂CH₂-Si(CH₃)₂-O-{Si(CH₃)₂-O}ₙ-Si(CH₃)₂-R²**
wobei -R² eine C₁- bis C₁₀-Alkylgruppe ist.

## Revendications

1. Procédé de modification des propriétés de surface d'un substrat polymère de tubulure de cathéter, comprenant :
la fourniture d'un fluide densifié comportant au moins un composé contenant du silicone et/ou du fluor dispersé ou dissous dans celui-ci ;
la fourniture d'un substrat polymère, où le substrat polymère est une tubulure de cathéter, où le substrat polymère a une surface capable d'incorporer l'au moins un composé contenant du silicone et/ou du fluor, et où l'incorporation de l'au moins un composé contenant du silicone et/ou du fluor dans la surface du substrat polymère diminue la tension superficielle de la surface ;
le positionnement du substrat polymère dans un réacteur ;
l'introduction dans le réacteur du fluide densifié ayant l'au moins un composé contenant du silicone et/ou du fluor dispersé ou dissous dans celui-ci ;
le maintien du réacteur dans des conditions de sorte que l'au moins un composé contenant du silicone et/ou du fluor soit transféré depuis le fluide densifié dans la surface du substrat polymère de sorte que l'au moins un composé contenant du silicone et/ou du fluor diminue la tension superficielle de ladite surface.

2. Procédé de la revendication 1, dans lequel le fluide densifié est introduit dans le réacteur dans des conditions subcritiques ; et, dans lequel les conditions subcritiques sont maintenues dans le réacteur pendant une durée contrôlée, après quoi les conditions dans le réacteur sont ajustées à des conditions supercritiques.

3. Procédé de la revendication 1, dans lequel le fluide densifié est introduit dans le réacteur dans des conditions supercritiques.

4. Procédé de l'une quelconque des revendications 1 à 3, dans lequel le composé contenant du silicone et/ou du fluor comprend un groupe fonctionnel polaire choisi dans le groupe constitué d'hydroxyle, amino, acide carboxylique, nitro, thio, urée et uréthane.

5. Procédé de l'une quelconque des revendications précédentes, dans lequel le fluide densifié comprend du dioxyde de carbone densifié.

6. Matériau obtenu ou pouvant être obtenu par l'un quelconque des procédés des revendications 1 à 5.

7. Procédé de l'une quelconque des revendications 1 à 5 dans lequel l'agent de modification de surface est un composé ayant la formule
-(CF₂)ₙ R ;
-(O-CF₂-CF₂)ₙ-R ; ou
-{CF(CF₃)-CF₂}ₙ-R ;
où :
n est égal à 1 à 100, et
R est -OH ;
-NH₂ ;
-COOH ;
-COOCH₃ ; ou
-OCOC (CH₃) =CH₂.

8. Procédé de l'une quelconque des revendications 1 à 5, dans lequel l'agent de modification de surface est un composé ayant la formule
-{S₁(CH₃)₂-0}ₙ-S₁(CH₃)₂-R¹
où :
n = 1 à 100, et
R¹ est -OH ;
-NH₂ ;
-COOH ;
-(CH₂CH₂O)ₘH, où m = 1 à 20 ;
-OCOC(CH₃)=CH₂ ; ou
-(CH₂)ₚO(CH₂)_{q}OH, où p et q chacun = 1 à 6, et peuvent être identiques ou différents l'un de l'autre.

9. Procédé de l'une quelconque des revendications 1 à 5, dans lequel l'agent de modification de surface est un composé ayant la formule HOCH₂CH₂OCH₂CH₂CH₂-Si (CH₃)₂-O-{Si(CH₃)₂-O}ₙ-Si(CH₃)₂-R² où -R² est un groupe alkyle en C₁ à C₁₀.
